# FASCICULE DE BREVET EUROPEEN

(11) **EP 0 792 862 B1**
(45) Date de publication et mention de la délivrance du brevet: **21.03.2001**
(21) Numéro de dépôt: 97400153.9
(22) Date de dépôt: 23.01.1997
(51) Int. Cl.: C07C 45/72, C07C 45/74, B01J 21/10

(54) **Procédé d'obtention de composés carbonyles beta hydroxy et/ou alpha-beta insaturés**
Verfahren zur Herstellung von beta hydroxy und/oder alpha-beta ungesättigten Carbonylverbindungen
Process for obtaining beta hydroxy and/or alpha-beta unsaturated carbonyl compounds

(30) Priorité: 29.02.1996 FR 9602551
(43) Date de publication de la demande: 03.09.1997
(73) Titulaire: Atofina, 92800 Puteaux (FR)
(72) Inventeur: Teissier, Remy, 69340 Francheville (FR); Fournier, Maurice, 73130 ST Etienne-de-Cuines (FR)

(56) Documents cités:
- EP-A- 0 095 783
- EP-A- 0 720 977
- WO-A-92/00266
- US-A- 5 254 743

## Description

La présente invention concerne un nouveau procédé d'obtention de composés carbonylés β hydroxy et/ou de composés carbonylés α - β insaturés. Plus particulièrement, elle a pour objet un procédé d'aldolisation et/ou d'aldolisation-crotonisation d'un aldéhyde et/ou d'une cétone.

Les procédés industriels basés sur la réaction de condensation aldolique des aldéhydes et des cétones font intervenir un catalyseur basique tel que les solutions diluées d'hydroxyde de potassium et d'hydroxyde de sodium. La séparation de ces catalyseurs à la fin de la réaction n'est cependant pas facile, et nécessite l'ajout des solutions acides notamment l'acide sulfurique pour convertir les catalyseurs en sels correspondants. De plus l'élimination de sels ainsi formés, nécessaire pour l'environnement est coûteuse pour l'industrie.

Pour pallier les inconvénients précités, l'utilisation des catalyseurs solides a été proposée. Ainsi l'oxyde de cuivre supporté sur une alumine y a été décrit dans le brevet US 4739122 pour catalyser la réaction de condensation aldolique croisée entre l'acétone et le butanal.

Le brevet US 5 144 089 divulgue un procédé de condensation aldolique en phase liquide, notamment la conversion du butanal en 2-éthyl 2-hexènal en présence d'un catalyseur solide. Le catalyseur est une solution solide d'oxyde de magnésium et d'oxyde d'aluminium dérivée d'une hydrotalcite et ayant une surface spécifique supérieure à 250 m²/g.

Le dioxyde de titane a également été décrit comme catalyseur de la réaction de condensation aldolique dans le procédé d'obtention des aldéhydes α-β insaturés (US 4316990).

Une catalyse sur résine échangeuse d'ions a également été mise en oeuvre mais sans grand succès car la résine ne peut pas supporter une température supérieure à 90° C. De plus la résine une fois désactivée, ne peut plus être régénérée.

Il a maintenant été trouvé que les inconvénients énoncés précédemment peuvent être surmontés en utilisant un catalyseur de formule générale (I) :

[(Mg²⁺)₁₋ₓ(Al³⁺)ₓ(OH⁻)₂]^{x+} [(OH⁻)ₓ ]^{x-} (H₂O)ₙ (I)

avec 0,20 ≤ x ≤ 0,33 et n < 1.

L'invention a donc pour objet un procédé d'obtention de composés carbonylés β hydroxy à l'exclusion de la diacétone alcool et/ou de composés carbonylés α - β insaturés. Ce procédé est caractérisé en ce que l'on met au moins un aldéhyde ou une cétone au contact d'un catalyseur solide de formule générale (I):

[(Mg²⁺)₁₋ₓ(Al³⁺)ₓ(OH⁻)₂]^{x+} [(OH⁻)ₓ ]^{x-} (H₂O)ₙ (I)

avec 0,20 ≤ x ≤ 0,33 et 0 < n < 1.

On utilise le plus souvent un aldéhyde ou une cétone ayant au moins un atome d'hydrogène sur le carbone en a de la fonction carbonyle.

Le procédé selon la présente invention consiste de préférence à faire réagir, en présence d'un catalyseur de formule générale ( I ), un aldéhyde de formule générale R¹- CHO, dans laquelle R¹ représente un radical alkyle linéaire ou ramifié contenant de 1 à 10 atomes de carbone, avec éventuellement au moins un composé choisi parmi un autre aldéhyde de formule générale R²- CHO, dans laquelle R² représente un atome d'hydrogène ou un radical alkyle linéaire ou ramifié ou cyclique contenant de 1 à 10 atomes de carbone ou un radical phényle ou un radical benzyle ou un radical aralkyle et/ou une cétone de formule générale R⁵-CO-R^{6,} dans laquelle R⁵ et R⁶ identiques ou différents représentent chacun un radical alkyle linéaire ou ramifié contenant de 1 à 10 atomes de carbone et pouvant être reliés entre eux pour former un cycle.

On préfère également faire réagir une cétone de formule générale R³-CO- R⁴ dans laquelle R³ et R⁴ identiques ou différents représentent chacun un radical alkyle linéaire ou ramifié contenant de 1 à 10 atomes de carbone, avec éventuellement au moins un composé choisi parmi un aldéhyde de formule générale R²- CHO, dans laquelle R² représente un atome d'hydrogène ou un radical alkyle linéaire ou ramifié ou cyclique contenant de 1 à 10 atomes de carbone ou un radical phényle ou un radical benzyle ou un radical aralkyle, et/ou une autre cétone de formule R⁵-CO-R^{6,} dans laquelle R⁵ et R⁶ identiques ou différents représentent chacun un radical alkyle linéaire ou ramifié contenant de 1 à 10 atomes de carbone et pouvant être reliés entre eux pour former un cycle, en présence d'un catalyseur de formule générale (I).

La présente invention a tout particulièrement pour objet un procédé d'aldolisation et/ou d'aldolisation-crotonisation d'au moins un aldéhyde ou une cétone (à l'exclusion du procédé d'aldolisation sélective de l'acétone en diacétone alcool) en présence d'un catalyseur de formule (I).

Avantageusement le procédé selon la présente invention comprend la mise en contact d'une cétone de formule générale R³-CO-R⁴ et d'un aldéhyde de formule générale R²- CHO avec un catalyseur de formule (I).

La cétone est de préférence choisie parmi celles dans lesquelles R³ ou R⁴ représente un radical méthyle comme notamment l'acétone, l'éthylméthylcétone ou la méthylpropylcétone. L'acétone est particulièrement préférée.

L'aldéhyde est avantageusement choisi parmi le méthanal, l'éthanal, le propanal, le butanal, l'isobutanal et le benzaldéhyde. De préférence on utilise le butanal ou l'isobutanal.

Avantageusement, le procédé comprend également la mise en contact d'un aldéhyde R¹ - CHO et éventuellement d'un autre aldéhyde R² CHO avec le catalyseur dont la formule (I) est donnée ci-dessus.

L'aldéhyde R¹ - CHO est avantageusement choisi parmi l'éthanal, le propanal, le butanal, l'isobutanal, le pentanal, l'hexènal et l'heptènal.

L'aldéhyde R² - CHO est avantageusement choisi parmi le méthanal, l'éthanal, le propanal, le butanal, l'isobutanal et le benzaldéhyde. De préférence on utilise le méthanal, le butanal et l'isobutanal.

Pour le procédé d'aldolisation et/ou d'aldolisation-crotonisation d'un aldéhyde, on utilise avantageusement l'éthanal, le butanal ou l'isobutanal. Le butanal est particulièrement préféré.

Sont avantageusement fabriqués selon le procédé de la présente invention, les composés carbonylés β- hydroxy et α - β insaturés tels que notamment l'oxyde de mésityle, le 2-éthyl 2-hexènal, la 5-méthyl 3-hexèn-2-one, la 3-heptèn-2-one,le 2,2',4-triméthyl 3-ol pentanal.

Les composés carbonylés α - β insaturés obtenus suivant le procédé de la présente invention peuvent être soumis soit à une hydrogénation sélective, en utilisant des catalyseurs connus, pour donner des composés carbonylés saturés correspondants comme les aldéhydes ou cétones ayant un poids moléculaire plus élevé que ceux ou celles qu'on met en oeuvre, soit à une hydrogénation totale pour donner les alcools saturés correspondants. Ces aldéhydes ou cétones ou alcools sont employés industriellement comme intermédiaire de synthèse ou comme solvant ou dans l'industrie des parfumeries.

Le procédé d'obtention des composés carbonylés β hydroxy et/ou des composés carbonylés α - β insaturés selon la présente invention peut être conduit à une température dans les limites aussi éloignées allant de 0° C à 200° C.

On opère le plus souvent à une température comprise entre 0° C et 140° C.

De préférence, on utilise une température comprise entre 0° C et 100° C.

La pression à laquelle on opère est telle que les réactifs (aldéhydes et/ou cétones) soient à l'état liquide à la température d'aldolisation ou d'aldolisation-crotonisation. Bien qu'il soit possible de travailler à pression supérieure à la pression atmosphérique, on préfère le plus souvent opérer à la pression atmosphérique.

Le procédé selon l'invention peut être mené aussi bien en continu qu'en discontinu. Industriellement, on préfère le procédé en continu.

Lorsqu'on travaille en continu, le procédé consiste à introduire au moins un aldéhyde et/ou une cétone à travers un lit fixe ou lit agité de catalyseur solide de formule général (I).

Les réactifs peuvent être introduits séparément ou préalablement mélangés en partie ou en totalité avant introduction dans le réacteur. Ils peuvent également être introduits dans le réacteur soit simultanément soit successivement. La façon d'opérer dépend de leur réactivité relative et du produit final souhaité.

D'une manière générale, lorsqu'on met en contact deux aldéhydes ou deux cétones ou une cétone et un aldéhyde ayant des réactivités différentes, en présence du catalyseur de formule générale (I), on utilise toujours un excès du composé le moins actif. Le rapport molaire de l'aldéhyde ou de la cétone le moins réactif sur l'aldéhyde ou la cétone le plus réactif est en général compris entre 1,2 et 12 et de préférence compris entre 1,5 et 8.

Lorsqu'on opère en discontinu, la quantité de catalyseur utilisée par rapport à la charge totale de réactifs introduits dans le réacteur est en général comprise entre 0,5 % et 20 %, et de préférence comprise entre 2% et 15 %.

Un rapport massique de catalyseur utilisé sur la charge totale de réactifs introduits, compris entre 3 % et 10 % est particulièrement préféré.

Le catalyseur de formule générale (I) :

[(Mg²⁺)₁₋ₓ(Al³⁺)ₓ(OH⁻)₂]^{x+} [(OH⁻)ₓ ]^{x-} (H₂O)ₙ (I)

ayant une valeur de n pouvant aller de 0,5 et 0,75 est le plus souvent utilisé dans le procédé selon la présente invention.

Avantageusement, on choisit un catalyseur de formule générale (I), ayant une valeur n égale à 0,81 - x, comme notamment la Meixnerite de formule :

[(Mg²⁺)_{0,75}(Al³⁺)_{0,25}(OH⁻)₂]^{0,25+} [(OH⁻)_{0,25}]^{0,25-} (H₂O)_{0,5}

Le catalyseur de formule générale (I) peut être préparé suivant la méthode décrite par G. Mascolo et O. Marino dans Mineralogical Magazine March 1980, Vol. 43 p. 619.

Cette méthode de préparation consiste à mettre en suspension du gel d'alumine et du MgO, obtenu par calcination de carbonate basique de magnésium à 650° C, dans de l'eau distillée, dans un récipient fermé en Téflon, sous agitation, pendant une semaine à 80 ± 1° C. La suspension est ensuite filtrée à l'abri du CO₂ et enfin le solide recueilli est séché sur silica gel.

Ce catalyseur peut également être préparé par hydratation d'un double oxyde de magnésium et d'aluminium Mg₁₋ₓ AlₓO₁₊ₓ en absence de CO₂. L'hydratation est effectuée par de l'eau soit en phase liquide soit en phase vapeur. Le double oxyde mixte peut être soit un produit commercial soit obtenu par calcination des hydrotalcites, ayant une valeur de x pouvant aller de 0,2 à 0,33, à une température inférieure à 800° C.

Après l'étape d'hydratation suivant l'une quelconque des méthodes décrites ci-dessus, le solide peut être séché soit par évaporation à pression réduite à une température inférieure à 60° C, soit par rinçage avec un solvant miscible à l'eau, comme par exemple l'acétone.

Pour préparer le catalyseur de formule générale (I), on choisit avantageusement un double oxyde commercial et de préférence celui de la société japonaise KYOWA, référencé KW 2000 et ayant une valeur de x voisine de 0,3.

Le double oxyde est le plus souvent hydraté en phase liquide et le solide ainsi obtenu est avantageusement rincé avec un solvant miscible à l'eau, et de préférence avec de l'acétone.

L'invention sera mieux comprise à partir des exemples non limitatifs suivants :

### Exemple 1

### Préparation de catalyseur.

On hydrate par de l'eau en phase liquide, le double oxyde mixte KW 2000 ayant les caractéristiques suivantes :
Formule chimique : 4,5 MgO. Al₂O₃ (x = 0,3077)
Volume massique apparente : 44 ml/10 g
Aspect : poudre blanche, fine sans odeur
BET = 172 m² /g
Taille moyenne de particule : 70 µm.
Propriété absorbante : absorbe au maximum 70 -80 parties d'eau pour 100 parties de KW 2000.

Ainsi 6 grammes de KW 2000 sont ajoutées sous agitation à 200 ml d'eau décarbonatée (eau permutée, puis bouillie). On laisse le mélange pendant 3 heures sous agitation, puis on sépare le solide. Le solide isolé est ensuite rincé plusieurs fois à l'acétone avant d'être conservé à l'abri de CO₂. On obtient 9 g de solide de formule générale (I) où x vaut 0,3077 et qui a une structure cristalline de type de l'hydrotalcite ou de la Meixnerite

### Exemple 2

Dans un réacteur agité thermostaté balayé par un courant d'azote, on introduit à température ambiante 4,9 g du catalyseur de l'exemple 1 suivi de 37 g d'acétone. Puis, on porte la charge sous agitation à 50° C en 30 minutes. On introduit ensuite en deux heures 100 g d'un mélange contenant 75 % en poids d'acétone et 25 % en poids d'isobutanal. Après la fin de l'introduction du mélange, on laisse réagir pendant une heure 30 minutes à 50° C. A la fin de la réaction, on filtre le catalyseur et on analyse la solution finale par chromatographie en phase gazeuse. Le chromatographe utilisé est un Perkin-Elmer 8420 équipé d'un détecteur FID, d'une colonne capillaire type DB 1701 de J & W de 25 nm de diamètre et de longueur 30 m. La température de l'injecteur est de 280° C et celle du détecteur de 175° C. L'injecteur comporte un split de 60 ml/min. Le four est programmé avec une pente de 3° C/min de la température initiale de 80° C vers la température finale de 200° C.

L'analyse chromatographique de la solution finale donne une conversion dl'isobutanal de 98 %, une sélectivité en 5 méthyl 4-ol 2-hexanone de 34 % et celle en 5 méthyl-3-hexèn-2-one de 45 %. La sélectivité totale en composés carbonylé β hydroxy et α-β insaturé est égale à 79 %.

### Exemple 3

On opère de manière identique à l'exemple 2 mais à la place d'isobutanal, on utilise le butanal. Les résultats obtenus à la fin de la réaction :
Conversion du butanal = 96 %
Sélectivité en 4-ol 2-heptanone = 41 %
Sélectivité en 3-heptèn-2-one = 30 %
Sélectivité totale en composés carbonylé β hydroxy et α - β insaturé = 71 %.

### Exemple 4 (comparatif)

On opère de manière identique à l'exemple 3 mais à la place du catalyseur de l'exemple 1, on utilise 4,9 g du double oxyde commercial KW 2000. Les résultats obtenus à la fin de la réaction :
Conversion du butanal = 7 %
Sélectivité en 4-ol 2-heptanone = 37 %
Sélectivité en 3-heptèn-2-one = 5 %
Sélectivité totale en composés carboxylé β hydroxy et α - β insaturé = 42 %

### Exemple 5 (comparatif)

On opère de manière identique à l'exemple 2 mais à la place du catalyseur de l'exemple 1, on utilise 4,9 g du double oxyde commercial KW 2000.

Les résultats obtenus à la fin de la réaction :
Conversion d'isobutanal = 7 %
Sélectivité en 5-méthyl 4-ol 2-hexanone = 40 %
Sélectivité en 5-méthyl 3-hexèn-2-one = 10 %.
Sélectivité totale en composés carbonylé β hydroxy et α - β insaturé = 50 %

### Exemple 6 (comparatif)

On opère de manière identique à l'exemple 2 mais à la place du catalyseur de l'exemple 1, on utilise 10 g de soude méthanolique 2N, soit 0,8 g de soude pure.

Les résultats obtenus à la fin de la réaction :
Conversion d'isobutanal = 99 %
Sélectivité en 5-méthyl 4-ol 2-hexanone = 13 %
Sélectivité en 5-méthyl 3-hexèn-2-one = 6%
Sélectivité totale en composés carbonylé β hydroxy et α-β insaturé = 19 %

### Exemple 7

Dans un réacteur agité thermostaté de 0,5 litre, balayé par un courant d'azote, on introduit à température ambiante 5 g du catalyseur de l'exemple 1 suivi de 100 g de butanal. Puis on porte la charge à 75° C en 30 minutes et on laisse sous agitation pendant 3 heures. A la fin de la réaction, on filtre le catalyseur et on analyse la solution finale par chromatographie en phase gazeuse de la même façon qu'à l'exemple 2.

On obtient une conversion du butanal de 77 %, une sélectivité en 2-éthyl-2-hexènal de 57 % et celle en 2-éthyl 3-ol hexanal de 10 %. La sélectivité totale en composés carbonylé β hydroxy et α-β insaturé est égale à 67 %.

### Exemple 8 comparatif

On opère de manière identique qu'à l'exemple 7, mais à la place du catalyseur de l'exemple 1, on utilise le double oxyde commercial KW 2000.

Au bout de 3 heures, on obtient une conversion du butanal de 8 % et une sélectivité en 2-éthyl-2-hexènal de 30 % et une sélectivité en 2-éthyl 3-ol hexanal de 50 %. La sélectivité totale en composés carbonylé β hydroxy et α-β insaturé est égale à 80 %.

## Revendications

1. Procédé d'obtention de composés carbonylés β hydroxy à l'exclusion de la diacétone alcool et/ou de composés carbonylés α - β insaturés caractérisé en ce que l'on met au moins un aldéhyde ou une cétone au contact d'un catalyseur solide de formule générale (I):
[(Mg²⁺)₁₋ₓ(Al³⁺)ₓ(OH⁻)₂]^{x+} [(OH⁻)ₓ]^{x-} (H₂O)ₙ (I)
avec 0,20 ≤ x ≤ 0,33 et 0 < n < 1.

2. Procédé selon la revendication 1 caractérisé en ce qu'au moins un aldéhyde ou une cétone a un atome d'hydrogène sur le carbone en a de la fonction carbonyle.

3. Procédé selon la revendication 1 ou 2 caractérisé en ce que l'on fait réagir un aldéhyde de formule générale R¹ - CHO, dans laquelle R¹ représente un radical alkyle linéaire ou ramifié contenant de 1 à 10 atomes de carbone avec éventuellement au moins un composé choisi parmi un autre aldéhyde de formule générale R²- CHO, dans laquelle R² représente un atome d'hydrogène ou un radical alkyle linéaire ou ramifié ou cyclique contenant de 1 à 10 atomes de carbone ou un radical phényle ou un radical benzyle ou un radical aralkyle et/ou une cétone de formule R⁵-CO-R⁶ dans laquelle R⁵ et R⁶ identiques ou différents représentent chacun un radical alkyle linéaire ou ramifié contenant de 1 à 10 atomes de carbone et pouvant être reliés entre eux pour former un cycle.

4. Procédé selon la revendication 1 ou 2 caractérisé en ce que l'on fait réagir une cétone de formule générale R³-CO-R⁴ dans laquelle R³ et R⁴ identiques ou différents représentent chacun un radical alkyle linéaire ou ramifié contenant de 1 à 10 atomes de carbone avec éventuellement au moins un composé choisi parmi un aldéhyde de formule générale R²-CHO, dans laquelle R² représente un atome d'hydrogène ou un radical alkyle linéaire ou ramifié ou cyclique contenant de 1 à 10 atomes de carbone ou un radical phényle ou un radical benzyle ou un radical aralkyle et/ou une autre cétone de formule R⁵-CO-R⁶ dans laquelle R⁵ et R⁶ identiques ou différents représentent chacun un radical alkyle linéaire ou ramifié contenant de 1 à 10 atomes de carbone et pouvant être reliés entre eux pour former un cycle.

5. Procédé selon la revendication 3 caractérisé en ce que l'aldéhyde de formule générale R¹- CHO est choisi parmi l'ethanal, le propanal, le butanal, l'isobutanal, le pentanal, l'hexanal et l'heptanal.

6. Procédé selon la revendication 5 caractérisé en ce que l'aldéhyde est le butanal ou l'isobutanal.

7. Procédé selon l'une des revendications 3 à 5 caractérisé en ce que l'aldéhyde de formule générale R²- CHO est choisi parmi le methanal, l'ethanal, le propanal, le butanal, l'isobutanal et le benzaldéhyde.

8. Procédé selon la revendication 7 caractérisé en ce que l'aldéhyde est le methanal, le butanal ou l'isobutanal.

9. Procédé selon la revendication 4, 7 où 8 caractérisé en ce que la cétone de formule générale R³-CO-R⁴ est choisi parmi l'acétone, l'éthylméthylcétone, le méthylpropylcétone.

10. Procédé selon la revendication 9 caractérisé en ce que la cétone est l'acétone.

11. Procédé selon l'une quelconque des revendications 1 à 10 caractérisé en ce que le catalyseur de formule générale (I) a une valeur de n allant de 0,5 à 0,75.

12. Procédé selon la revendication 11 caractérisé en ce que la valeur de n est égale à 0,81 - x.

13. Procédé selon l'une quelconque des revendications 1 à 12 caractérisé en ce que l'on opère à une température comprise entre 0 et 200° C.

14. Procédé selon la revendication 13 caractérisé en ce que la température est comprise entre 0 et 140° C.

15. Procédé selon la revendication 14 caractérisé en ce que la température est comprise entre 0 et 100° C.

16. Procédé d'obtention du 4-ol 2-heptanone et/ou 3 heptèn-2-one selon l'une quelconque des revendications 1 à 15.

17. Procédé d'obtention du 5 méthyl 4-ol 2-hexanone et/ou 5 méthyl 3-hexèn-2-one selon l'une quelconque des revendication 1 à 15.

18. Procédé d'obtention du 2-éthyl 3-ol hexanal et/ou 2-éthyl 2-hexènal selon l'une quelconque des revendications 1 à 3 ; 5 à 8 et 11 à 15.

## Claims

1. Process for the production of β-hydroxy carbonyl compounds, with the exception of diacetone alcohol, and/or of α,β-unsaturated carbonyl compounds, characterized in that at least one aldehyde or one ketone is brought into contact with a solid catalyst of general formula (I):
[(Mg²⁺)₁₋ₓ(Al³⁺)ₓ(OH⁻)₂]^{x+}[(OH⁻)ₓ]^{x-}(H₂O)ₙ (I)
with 0.20 ≤ x ≤ 0.33 and 0 < n < 1.

2. Process according to Claim 1, characterized in that at least one aldehyde or one ketone has a hydrogen atom on the carbon α to the carbonyl functional group.

3. Process according to Claim 1 or 2, characterized in that an aldehyde of general formula R¹-CHO, in which R¹ represents a linear or branched alkyl radical comprising from 1 to 10 carbon atoms, is reacted with optionally at least one compound chosen from another aldehyde of general formula R²-CHO, in which R² represents a hydrogen atom or a linear or branched or cyclic alkyl radical comprising from 1 to 10 carbon atoms or a phenyl radical or a benzyl radical or an aralkyl radical, and/or a ketone of formula R⁵-CO-R⁶, in which R⁵ and R⁶, which are identical or different, each represent a linear or branched alkyl radical comprising from 1 to 10 carbon atoms which can be connected to one another to form a ring.

4. Process according to Claim 1 or 2, characterized in that a ketone of general formula R³-CO-R⁴, in which R³ and R⁴, which are identical or different, each represent a linear or branched alkyl radical comprising from 1 to 10 carbon atoms, is reacted with optionally at least one compound chosen from an aldehyde of general formula R²-CHO, in which R² represents a hydrogen atom or a linear or branched or cyclic alkyl radical comprising from 1 to 10 carbon atoms or a phenyl radical or a benzyl radical or an aralkyl radical, and/or another ketone of formula R⁵-CO-R⁶, in which R⁵ and R⁶, which are identical or different, each represent a linear or branched alkyl radical comprising from 1 to 10 carbon atoms which can be connected to one another to form a ring.

5. Process according to Claim 3, characterized in that the aldehyde of general formula R¹-CHO is chosen from ethanal, propanal, butanal, isobutanal, pentanal, hexanal and heptanal.

6. Process according to Claim 5, characterized in that the aldehyde is butanal or isobutanal.

7. Process according to one of Claims 3 to 5, characterized in that the aldehyde of general formula R²-CHO is chosen from methanal, ethanal, propanal, butanal, isobutanal and benzaldehyde.

8. Process according to Claim 7, characterized in that the aldehyde is methanal, butanal or isobutanal.

9. Process according to Claim 4, 7 or 8, characterized in that the ketone of general formula R³-CO-R⁴ is chosen from acetone, ethyl methyl ketone or methyl propyl ketone.

10. Process according to Claim 9, characterized in that the ketone is acetone.

11. Process according to any one of Claims 1 to 10, characterized in that the catalyst of general formula (I) has a value of n ranging from 0.5 to 0.75.

12. Process according to Claim 11, characterized in that the value of n is equal to 0.81 - x.

13. Process according to any one of Claims 1 to 12, characterized in that it is carried out at a temperature of between 0 and 200°C.

14. Process according to Claim 13, characterized in that the temperature is between 0 and 140°C.

15. Process according to Claim 14, characterized in that the temperature is between 0 and 100°C.

16. Process for the production of 4-hydroxy-2-heptanone and/or 3-hepten-2-one according to any one of Claims 1 to 15.

17. Process for the production of 5-methyl-4-hydroxy-2-hexanone and/or 5-methyl-3-hexen-2-one according to any one of Claims 1 to 15.

18. Process for the production of 2-ethyl-3-hydroxyhexanal and/or 2-ethyl-2-hexenal according to any one of Claims 1 to 3, 5 to 8 and 11 to 15.

## Patentansprüche

1. Verfahren zur Herstellung von β-Hydroxycarbonylverbindungen unter Ausschluß von Diacetonalkohol und/oder von α-β-ungesättigten Carbonylverbindungen, dadurch gekennzeichnet, daß man mindestens einen Aldehyd oder ein Keton mit einem festen Katalysator mit der allgemeinen Formel (I) zusammenbringt:
[(Mg²⁺)₁₋ₓ (Al³⁺)ₓ (OH⁻)₂]^{x+} [(OH⁻)ₓ]^{x-} (H₂O)ₙ (I)
mit 0,20 ≤ x ≤ 0,33 und 0 < n < 1.

2. Verfahren nach Anspruch 1, dadurch gekennzeichnet, daß mindestens ein Aldehyd oder ein Keton ein Wasserstoffatom an dem Kohlenstoff in α-Stellung zur Carbonylgruppe trägt.

3. Verfahren nach Anspruch 1 oder 2, dadurch gekennzeichnet, daß man einen Aldehyd der allgemeinen Formel R¹-CHO, in der R¹ einen linearen oder verzweigten Alkylrest mit 1 bis 10 Kohlenstoffatomen bedeutet, mit gegebenenfalls mindestens einer Verbindung reagieren läßt, die aus einem anderen Aldehyd der allgemeinen Formel R²-CHO gewählt ist, in der R² ein Wasserstoffatom oder einen linearen oder verzweigten oder cyclischen Alkylrest mit 1 bis 10 Kohlenstoffatomen oder einen Phenylrest oder einen Benzylrest oder einen Aralkylrest bedeutet, und/oder einem Keton der Formel R⁵-CO-R⁶, in der die gleichen oder verschiedenen R⁵ und R⁶ ein jedes einen linearen oder verzweigten Alkylrest mit 1 bis 10 Kohlenstoffatomen bedeuten und beide miteinander verbunden sein können, um einen Ring zu bilden.

4. Verfahren nach Anspruch 1 oder 2, dadurch gekennzeichnet, daß man ein Keton der allgemeinen Formel R³-CO-R⁴, in der die gleichen oder verschiedenen R³ und R⁴ ein jedes einen linearen oder verzweigten Alkylrest mit 1 bis 10 Kohlenstoffatomen bedeuten, mit gegebenenfalls mindestens einer Verbindung reagieren läßt, die aus einem Aldehyd der allgemeinen Formel R²-CHO gewählt ist, in der R² ein Wasserstoffatom oder einen linearen oder verzweigten oder cyclischen Alkylrest mit 1 bis 10 Kohlenstoffatomen oder einen Phenylrest oder einen Benzylrest oder einen Aralkylrest bedeutet, und/oder einem anderen Keton der Formel R⁵-CO-R⁶, in der die gleichen oder verschiedenen R⁵ und R⁶ ein jedes einen linearen oder verzweigten Alkylrest mit 1 bis 10 Kohlenstoffatomen bedeuten und miteinander verbunden sein können, um einen Ring zu bilden.

5. Verfahren nach Anspruch 3, dadurch gekennzeichnet, daß der Aldehyd der allgemeinen Formel R¹-CHO aus Ethanal, Propanal, Butanal, Isobutanal, Pentanal, Hexanal und Heptanal gewählt ist.

6. Verfahren nach Anspruch 5, dadurch gekennzeichnet, daß der Aldehyd Butanal oder Isobutanal ist.

7. Verfahren nach einem der Ansprüche 3 bis 5, dadurch gekennzeichnet, daß der Aldehyd der allgemeinen Formel R²-CHO aus Methanal, Ethanal, Propanal, Butanal, Isobutanal und Benzaldehyd gewählt ist.

8. Verfahren nach Anspruch 7, dadurch gekennzeichnet, daß der Aldehyd Methanal, Butanal oder Isobutanal ist.

9. Verfahren nach Anspruch 4, 7 oder 8, dadurch gekennzeichnet, daß das Keton der allgemeinen Formel R³-CO-R⁴ aus Aceton, Ethylmethylketon und Methylpropylketon gewählt ist.

10. Verfahren nach Anspruch 9, dadurch gekennzeichnet, daß das Keton Aceton ist.

11. Verfahren nach irgendeinem der Ansprüche 1 bis 10, dadurch gekennzeichnet, daß der Katalysator der allgemeinen Formel (I) einen Wert für n von 0,5 bis 0,75 hat.

12. Verfahren nach Anspruch 11, dadurch gekennzeichnet, daß der Wert für n 0,81 - x ist.

13. Verfahren nach irgendeinem der Ansprüche 1 bis 12, dadurch gekennzeichnet, daß man bei einer Temperatur zwischen 0 und 200 °C arbeitet.

14. Verfahren nach Anspruch 13, dadurch gekennzeichnet, daß die Temperatur zwischen 0 und 140 °C liegt.

15. Verfahren nach Anspruch 14, dadurch gekennzeichnet, daß die Temperatur zwischen 0 und 100 °C liegt.

16. Verfahren zur Herstellung von 4-ol-2-Heptanon und/oder 3-Hepten-2-on nach irgendeinem der Ansprüche 1 bis 15.

17. Verfahren zur Herstellung von 5-Methyl-4-ol-2-hexanon und/oder 5-Methyl-3-hexen-2-on nach irgendeinem der Ansprüche 1 bis 15.

18. Verfahren zur Herstellung von 2-Ethyl-3-ol-hexanal und/oder 2-Ethyl-2-Hexenal nach irgendeinem der Ansprüche 1 bis 3; 5 bis 8 und 11 bis 15.
